# EUROPEAN PATENT APPLICATION

(11) **EP 1 738 772 A1**
(43) Date of publication of application: **03.01.2007**
(21) Application number: 05727699.0
(22) Date of filing: 28.03.2005
(51) Int. Cl.: A61K 48/00, A61K 31/7105, A61K 31/711, A61K 35/76, A61P 35/00, A61P 35/02

(54) **MICRO RNA INHIBITING THE EXPRESSION OF WT1 GENE AND UTILIZATION OF THE SAME**

(30) Priority: 29.03.2004 JP 2004096877
(71) Applicant: Sugiyama, Haruo, Minoo-shi, Osaka 562-0036 (JP)
(72) Inventor: SUGIYAMA, Haruo, Minoo-shi, Osaka 5620036 (JP); OJI, Yusuke, Suita-shi, Osaka 5640051 (JP)
(74) Representative: Vossius & Partner
(86) International application number: PCT/JP2005/005790
(87) International publication number: WO 2005/092393

(57) **Abstract**

It was found that miRNA that targets the vicinity of the WT1 gene stop codon not only suppresses the expression of the WT1 gene, but also exhibits a marked effect of suppressing cell growth in cancer cell lines.

## Description

### Technical Field

The present invention relates to microRNA that suppresses the expression of WT1 gene and uses thereof. In particular, the present invention relates to suppression of cell growth using such microRNA.

### Background Art

Wilms tumor gene (WT1 gene) is a gene encoding a zinc-finger transcription factor. WT1 gene is known to have four isoforms distinguished by the presence or absence of 17 amino acids (17AA) inserted at the 5'-side site of the two alternative splicing sites of the gene, as well as by the presence or absence of three amino acid residues between zinc fingers 3 and 4.

Wilms tumor gene (WT1 gene) was isolated as a causative gene of pediatric kidney tumor (Non-patent Documents 1 and 2). Since some deletions and mutations in this gene have been found in Wilms tumor, it has been believed that this gene is a tumor suppressor gene.

However, a number of reports by the present inventors suggest that WT1 gene exerts an oncogene-like function rather than functions as a tumor suppressor gene. It has been revealed that almost all leukemia cells express high levels of the nonmutated wild type WT1 gene, and the expression level in leukemia is reciprocally correlated with the prognosis of patients (Non-patent Documents 3 and 4); antisense WT1 DNA specifically suppresses the growth of leukemia cells (Non-patent Document 5); forced expression of the WT1 gene results in suppression of the differentiation of mouse normal bone marrow precursor cells and bone marrow precursor cell line 32D C 13 into neutrophils, and the cells began proliferating as a result (Non-patent Document 6); *etc.* These findings suggest that the WT1 gene is involved in the leukemogenic conversion of hematopoietic cells. The present inventors have also reported that the wild type WT1 gene is expressed at high levels in various types of solid cancers (Non-patent Documents 7 to 14).

The present inventors believe that the WT1 gene would be useful for the development of tumor-specific molecular target therapy if expression of the gene can be efficiently suppressed. To date, no tumor-specific molecular target therapy that targets WT1 has been known.
[Non-patent Document 1] Call KM, et al.: Isolation and characterization of a zinc finger polypeptide gene at the human chromosome 11 Wilms' tumor locus. Cell 60: 509, 1990
a [Non-patent Document 2] Gessler M, et al.: Homozygous deletion in Wilms tumours of zinc-finger gene identified by chromosome jumping. Nature 343 '. 774, 1990
[Non-patent Document 3] Inoue K, et al.: WT1 as a new prognostic factor and a new marker for the detection of minimal residual disease in acute leukemia. Blood 84: 3071, 1994
[Non-patent Document 4] Inoue K, et a/.: Aberrant overexpression of the Wilms tumor gene (WT1) in human leukemia. Blood 89: 1405, 1997
[Non-patent Document 5] Yamagami T, Sugiyama H, Inoue K, Ogawa H, Tatekawa T, Hirata M, Kudoh T, Akiyama T, Murakami A, Maekawa T. Growth inhibition of human leukemic cells by WT1 (Wilms tumor gene) antisense oligodeoxynucleotides: implications for the involvement of WT1 in leukemogenesis. Blood. 1996 Apr 1;87(7):2878-84.
[Non-patent Document 6] Inoue K, et al.: Wilms' tumor gene (WT1) competes with differentiation-inducing signal in hematopoietic progenitor cells. Blood 91:2969, 1998 [Non-patent Document 7] Oji, Y., Ogawa, H., Tamaki, H., Oka, Y., Tsuboi, A., Kim, E.H., Soma, T., Tatekawa, T., Kawakami, M., Asada, M., Kishimoto, T., and Sugiyama, H. Expression of the Wilms' tumor gene WT1 in solid tumors and its involvement in tumor cell growth. Japanese Journal of Cancer Research, 90: 194-204, 1999.
[Non-patent Document 8] Oji, Y, Miyoshi, S., Maeda, H., Hayashi, S., Tamaki, H., Nakatsuka, S., Yao, M., Takahashi, E., Nakano, Y., Hirabayashi, H., Shintani, Y., Oka, Y, Tsuboi, A., Hosen, N., Asada, M., Fujioka, T., Murakami, M., Kanato, K., Motomura, M., Kim, E.H., Kawakami, M., Ikegame, K., Ogawa, H., Aozasa, K., Kawase, I., and Sugiyama, H.: Overexpression of the Wilms' tumor gene WT1 in de novo lung cancers. International Journal of Cancer, 100: 304-308, 2002.
[Non-patent Document 9] Ueda, T., Oji, Y., Naka, N., Nakano, Y, Takahashi, E., Koga, S., Asada, M., Ikeba, A., Nakatsuka, S., Abeno, S., Hosen, N., Tomita, Y, Aozasa, K., Tamai, N., Myoui, A., Yoshikawa, H., and Sugiyama, H.: Overexpression of the Wilms' tumor gene WT1 in human bone and soft-tissue sarcomas. Cancer Science, 94: 271-276, 2003.
[Non-patent Document 10] Oji, Y, Inohara, H., Nakazawa, M., Nakano, Y, Akahani, S., Nakatusuka, S., Koga, S., Abeno, S., Honjo, Y., Yamamoto, Y, Iwai, S., Yoshida, K., Oka, Y, Ogawa, H., Yoshida, J., Aozasa, K., Kubo, T., and Sugiyama, H.: Overexpression of the Wilms' tumor gene WT1 in head and neck squamous cell carcinoma. Cancer Science, 94: 523-529, 2003. [Non-patent Document 11] Oji, Y, Miyoshi, Y, Koga, S., Nakano, Y, Ando, A., Nakatuska, S., Ikeba, A., Takahashi, E., Sakaguchi, N., Yokota, A., Hosen, N., Ikegame, K., Kawakami, M., Tsuboi, A., Oka, Y, Ogawa, H., Aozasa, K., Noguchi, S., and Sugiyama, H. Overexpression of the Wilms' tumor gene WT1 in primary thyroid cancer. Cancer Science, 94: 606-611, 2003.
[Non-patent Document 12] Oji, Y, Yamamoto, H., Nomura, M., Nakano, Y, Ikeba, A., Nakatsuka, S., Abeno, S., Kiyotoh, E., Jomgeow, T., Sekimoto, M., Nezu, R., Yoshikawa, Y., Inoue, Y, Hosen, N., Kawakami, M., Tsuboi, A., Oka, Y, Ogawa, H., Souda, S., Aozasa, K., Monden, M., and Sugiyama, H. Overexpression of the Wilms' tumor gene WT1 in colorectal adenocarcinoma. Cancer Science, 94: 712-717, 2003.
[Non-patent Document 13] Oji, Y., Miyoshi, S., Takahashi, E., Koga, S., Nakano, Y, Shintani, Y, Hirabayashi, H., Matsumura, A., Iuchi, K., Ito, K., Kishimoto, Y., Tsuboi, A., Ikegame, K., Hosen, N., Oka, Y, Ogawa, H., Maeda, H., Hayashi, S., Kawase, I., and Sugiyama, H. Absence of mutations in the Wilms' tumor gene WT1 in de novo non-small cell lung cancers. Neoplasma, 51:17-20, 2004.
[Non-patent Document 14] Oji, Y, Miyoshi, Y, Kiyotoh, E., Koga, S., Nakano, Y, Ando, A., Hosen, N., Tsuboi, A., Kawakami, M., Ikegame, K., Oka, Y, Ogawa, H., Noguchi, S., and Sugiyama, H. Absence of mutations in the Wilms' tumor gene WT1 in primary breast cancer. Jpn J Clin Oncol, 34:74-7, 2004.
[Non-patent Document 15] Experimental Medicine, Vol.22 No.4 (March) 494-499, 2004 [Non-patent Document 16] Oji Y, Suzuki T, Nakano Y, Maruno M, Nakatsuka S, Jomgeow T, Abeno S, Tatsumi N, Yokota A, Aoyagi S, Nakazawa T, Ito K, Kanato K, Shirakata T, Nishida S, Hosen N, Kawakami M, Tsuboi A, Oka Y, Aozasa K, Yoshimine T, Sugiyama H: Overexpression of the Wilms' tumor gene WT1 in primary astrocytic tumors. Cancer Sci. 95:822-7, 2004.
[Non-patent Document 17] Oji Y, Yano M, Nakano Y, Abeno S, Nakatsuka S, Ikeba A, Yasuda T, Fujiwara Y, Takiguchi S, Yamamoto H, Fujita S, Kanato K, Ito K, Jomgeow T, Kawakami M, Tsuboi A, Shirakata T, Nishida S, Hosen N, Oka Y, Aozasa K, Monden M, Sugiyama H.: Overexpression of the Wilms' tumor gene WT1 in esophageal cancer. Anticancer Res. 24:3103-8, 2004.
[Non-patent Document 18] Oji Y, Nakamori S, Fujikawa M, Nakatsuka S, Yokota A, Tatsumi N, Abeno S, Ikeba A, Takashima S, Tsujie M, Yamamoto H, Sakon M, Nezu R, Kawano K, Nishida S, Ikegame K, Kawakami M, Tsuboi A, Oka Y, Yoshikawa K, Aozasa K, Monden M, Sugiyama H.: Overexpression of the Wilms' tumor gene WT1 in pancreatic ductal adenocarcinoma. Cancer Sci. 95:583-7, 2004.

### Disclosure of the Invention

An objective of the present invention is to provide molecules that can efficiently suppress the expression of WT1 gene and to suppress cell growth using these molecules. In particular, the present invention provides microRNA as a molecule that suppresses the expression of WT1 gene.

To achieve the above objective, the present inventors conceived the idea of using microRNA to suppress the expression of WT1 in cancer cells. The inventors searched a database for microRNAs that would act on the WT1 mRNA sequence, and selected microRNA (miR) 115 as a candidate. Under the present technical situation, it is not easy to identify targets of microRNA because the binding of microRNA to its target mRNA is incomplete (Non-patent Document 15). However, as a result of dedicated studies, the present inventors discovered that the WT1 gene-targeting microRNA not only suppresses the expression of WT1 gene but also exhibits a marked effect of cell growth suppression in cancer cell lines. In addition, the inventors constructed a vector containing a potential target sequence inserted on the 3' side of a GFP protein and analyzed the effect ofmiR115 on the cellular expression of the GFP protein, and demonstrated that miR115 suppresses the expression of WT1 protein *via* the potential target sequence of WT1 mRNA. Thus, the present invention relates to suppression of cell growth using WT1-targeting microRNA, and more specifically, provides the following inventions:
[1] a cell growth-suppressing agent comprising any one of (a) to (c) as an active ingredient:
   (a) a single-stranded RNA complementary to a transcript of WT1 gene;
   (b) a DNA encoding the RNA of (a); and
   (c) a vector carrying the DNA of (b) as an insert;
[2] the cell growth-suppressing agent of [1], wherein the single-stranded RNA is complementary to the nucleotide sequence of the WT1 gene transcript shown in SEQ ID NO: 1;
[3] the cell growth-suppressing agent of [1] or [2], wherein the single-stranded RNA comprises the nucleotide sequence of SEQ ID NO: 2;
[4] a cell differentiation-inducing agent or cell death-inducing agent comprising any one of (a) to (c) as an active ingredient:
   (a) a double-stranded RNA comprising an RNA complementary to a transcript of WT1 gene and an RNA complementary to the RNA;
   (b) a DNA encoding the double-stranded RNA of (a); and
   (c) a vector carrying the DNA of (b) as an insert;
[5] the agent of [4], wherein the single-stranded RNA is complementary to the nucleotide sequence of the WT1 gene transcript shown in SEQ ID NO: 1; and
[6] the agent of [4] or [5], wherein the single-stranded RNA comprises the nucleotide sequence of SEQ ID NO: 2.

### Brief Description of the Drawings

Fig. 1 is a diagram showing complementarity between human miR-115 (SEQ ID NO: 2) and the target region of WT1 mRNA (SEQ ID NO: 1).
Fig. 2 is a graph showing a time course of the cell growth-suppressing effect of miR-115 on AZ-521 cells.
Fig. 3 is a graph showing the concentration dependency of the cell growth-suppressing effect of miR-115 in AZ-521 cells.
Fig. 4 is a photograph showing the suppression of WT1 protein expression by miR-115.
Fig. 5 is a graph showing that forced expression of WT1 blocked the cell growth-suppressing effect of miR-115 on AZ-521 cells.
Fig. 6 is a diagram showing the structures of GFP expression vectors containing a potential miR115 target sequence insert.
Fig. 7 is a photograph showing the suppression of GFP protein by miR115 *via* the potential miR115 target sequence.

### Best Mode for Carrying out the Invention

The present invention relates to microRNA that binds to transcripts of the WT1 gene. In general, microRNA is understood as small noncoding RNAs that induce gene silencing and act on mRNA sequences. MicroRNA (miRNA) was reported for the first time in 1993 by the group of Amboros *et al.* They reported that nematode lin-4, a noncoding RNA, bound to the 3'-UTR of the lin-14 or lin-28 gene and suppressed the expression of the gene at the translational level. After that, let-7, a nematode miRNA consisting of 21 nucleotides, was reported. Studies on let-7 suggest the possibility that a single miRNA regulates multiple target mRNAs. Today, it is known that miRNA exists in a broad range of animals and plants, and 300 types or more have been reported.

The expression process and function of miRNA are understood as follows. Precursor RNA comprising several tens to several hundreds of nucleotides is transcribed from a miRNA-encoding gene. The precursor RNA is processed into stem-loop RNA called pre-miRNA by nuclear ribonuclease. The pre-miRNA forms a complex with a transport protein and is transported outside the nucleus. The pre-miRNA is then processed into mature functional miRNA by Dicer. The mature miRNA is incorporated into the protein complex miRNP. It is believed that the resulting miRNP complex binds to a target mRNA that is partially complementary to the miRNA, and then suppresses the translation. It has also been reported that some miRNAs are perfectly complementary to particular mRNA sequences and function as siRNA. Like siRNA, such miRNAs are thought to suppress gene expression through sequence-specific cleavage of their target mRNA sequences, while ordinary miRNAs suppress gene expression through translation inhibition.

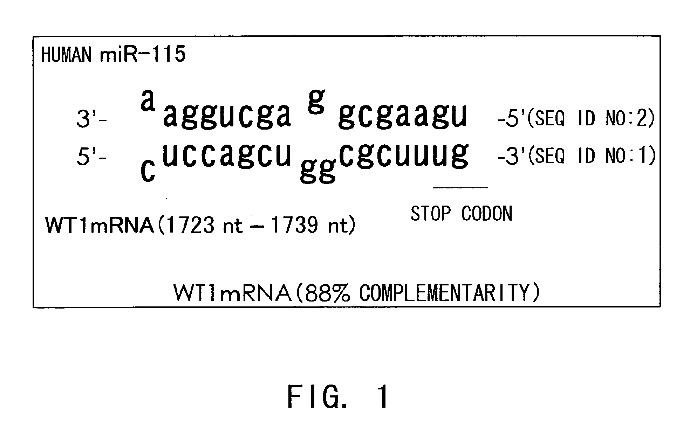

The microRNAs of the present invention are single-stranded RNAs that are complementary to the transcript of the target WT1 gene. Herein, "complementary" does not necessarily mean perfect complementarity. The binding between a microRNA of the present invention and a transcript of the target WT gene may comprise unpaired portions, such as mismatches (the corresponding nucleotides are not complementary) and bulges (the corresponding nucleotides do not exist on the other strand) (see Fig. 1).

The sequence of the transcript of WT1 gene, which is the target of microRNA of the present invention, is not limited as long as the microRNA can bind to the sequence and produce the effect of suppressing gene expression. An example of a preferred target sequence is the sequence of SEQ ID NO: 1. The sequence of SEQ ID NO: 1 corresponds to the region immediately before the stop codon, and is shared by the four isoforms of WT1 gene. An isoform of the WT1 gene is shown in SEQ ID NO.: 3. The SEQ ID NO: 1 target sequence corresponds to positions 1723 to 1739 in the WT1 gene sequence SEQ ID NO: 3, and the stop codon is located at positions 1738 to 1740. The start codon is located at positions 391 to 393.

miR-115 was used as a microRNA targeting the nucleotide sequence of SEQ ID NO: 1 in the Examples described herein. The nucleotide sequence of miR-115 is shown in SEQ ID NO: 2. miR-115 was identified by Dreyfuss *et al.* as a microRNA that forms the protein complex miRNP (ribonucleoprotein) with component proteins eIF2C, Gemin3, and Gemin4. It is known that Gemin3 and Gemin4, as well as Gemin2 and Gemin5, form an SMN complex together with "Survival of Motor Neuron" (SMN), a causative protein of spinal muscular atrophy. The NCBI GenBank number of miR-115 isAF480513.

There is no limitation on the length of the microRNAs of the present invention as long as the microRNAs have the effect of suppressing the expression of WT1 gene. The length is preferably 25 nucleotides or shorter, more preferably 14 to 18 nucleotides, and most preferably 16 nucleotides.

The miRNA of the present invention can be prepared based on a target sequence selected from the nucleotide sequence of 3'-UTR.

The miRNA of the present invention can be appropriately prepared by chemical synthesis or the like based on a target sequence selected from the nucleotide sequence of the WT1 gene transcript.

The miRNA of the present invention may be administered directly to a living body. The miRNA may also be expressed in the body by administering a DNA encoding the miRNA to the body. When expressing the miRNA in the body, it is possible to use viral vectors, for example, retrovirus, adenovirus, and Sendai virus, and non-viral vectors, such as liposome. The administration methods include, for *example, in vivo* and *ex vivo* methods.

Combined with an appropriate compounding ingredient, the miRNA of the present invention, DNA encoding the miRNA, and a vector carrying the DNA as an insert can be used as a cell growth-suppressing agent, cell differentiation-inducing agent, or cell death-inducing agent. Once the agent is introduced into cells using known transfection reagents or the like, it can produce the effect of cell growth suppression, induction of cell differentiation or cell death, or the like by translation inhibition or cleavage of the WT1 transcript. Cells for which the agents of the present invention are expected to be effective are cells that express the WT1 gene. Cancer cells express the WT1 gene at high levels. Specifically, the types of cancer cells that express WT1 at high levels include, for example, human leukemia, colon cancer, lung cancer, breast cancer, head and neck squamous carcinoma, esophageal cancer, stomach cancer, thyroid cancer, osteosarcoma and soft tissue sarcoma, ovarian cancer, uterine cancer, kidney cancer, pancreatic cancer, and glioblastoma. On the other hand, normal cells express WT1 at very low levels. Thus, the agents of the present invention can be effective not only for research purposes, but also as cancer therapy agents for human and other mammals, in particular for the cancers listed above. Such cancer therapy agents of the present invention are expected to be specific to cancer cells and give little damage to normal cells.

In preparing the agents of the present invention, pharmaceutically acceptable compounding ingredients may be combined. Such pharmaceutically acceptable compounding ingredients include, for example, detergents, excipients, coloring agents, flavoring agents, preservatives, stabilizers, buffers, suspending agents, isotonizing agents, binding agents, disintegrators, lubricants, fluidizing agents, and corrigents, but are not limited thereto. It is also possible to use other conventional carriers as needed. The dosage form of the preparation described above includes, for example, oral preparations, such as tablets, epipastics, pills, powders, granules, grains, soft and hard capsules, film-coated preparations, pellets, sublingual preparations, and pastes; and parenteral preparations, such as injections, suppositories, transdermal preparations, ointments, plasters, and liquid for external use. Those skilled in the art can select an optimal dosage form depending on the administration route, subject, and the like.

All the prior art documents cited herein are incorporated herein by reference.

### Examples

The present invention will be explained in more detail below with reference to Examples, but is not to be construed as being limited thereto.

### [Example 1] Selection of microRNA

A database (NCBI GenBank) was searched for potential microRNAs targeting the sequence immediately before the stop codon and up to the 3'-UTR of the WT1 mRNA. MicroRNA (miR) 115 was selected as a candidate.

### [Example 2] Preparation of microRNA

RNAs were custom-synthesized by Japan Bio Service (JBioS). The RNAs were dissolved in RNase-free water at a concentration of 100 µM. The solutions were aliquoted, and frozen and stored at -80°C prior to use. The sequences of the synthesized RNAs are shown below.
mir-115: 5' -uga agc gga gcu gga a-3' (SEQ ID NO: 2)
Luciferase AS: 5' -ucg aag uau ucc gcg uac guu -3' (SEQ ID NO: 4)

### [Example 3] Growth suppression of AZ-521 cells by miR-115

Cells of the gastric cancer cell line AZ-521 that expresses the WT1 gene at high levels were cultured in Dulbecco's Modified Medium (DMEM) containing 10% FBS. The cells were treated with the microRNA by the following procedure. The AZ-521 cells were trypsinized and adjusted to 1.2 x 10⁵ cells/2 ml. Then, the cells were plated in 6-well plates. After 24 hours, the RNA (final conc. 2 µM) was introduced into the cells using RNAi Fect (QIAGEN). The cell count was determined using a cytometer after trypsinization.

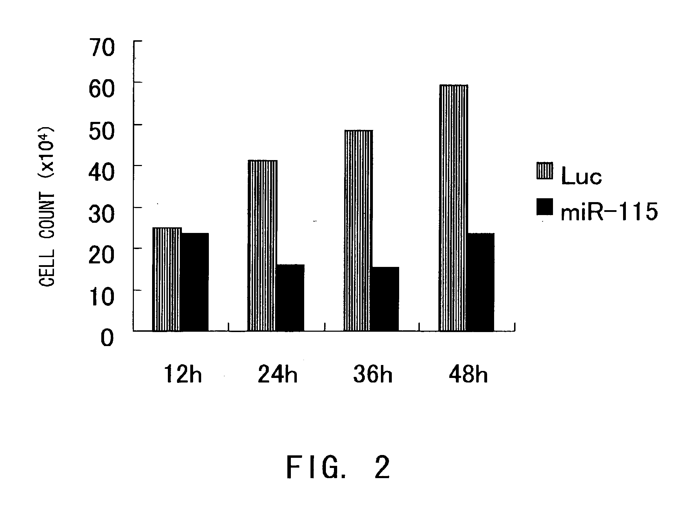

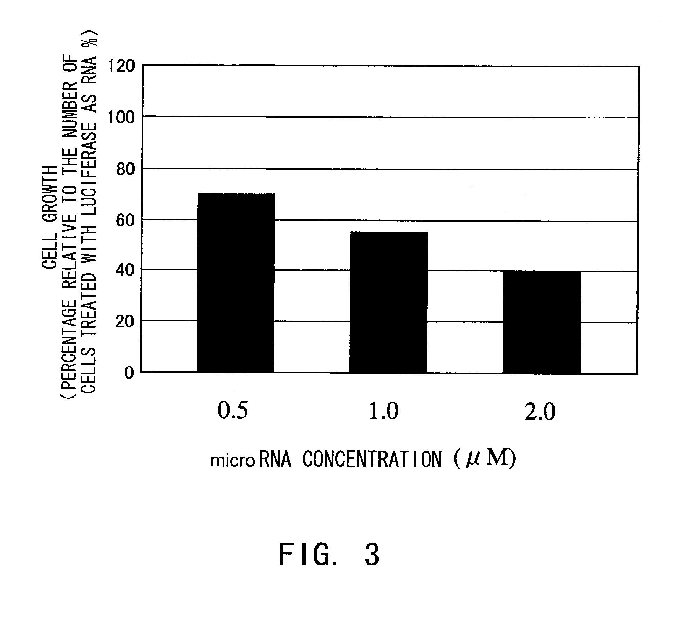

When AZ-521 cells were treated with miR-115 (final conc. 2 µM), the growth of AZ-521 cells was significantly suppressed as compared with control RNA (Fig. 2). Next, AZ-521 cells were treated for 48 hours with 0.5, 1.0, and 2.0 µM miR-115, and the cell growth-suppressing effect of miR-115 was analyzed. The effect was found to be dependent on the miR-115 concentration (Fig. 3).

### [Example 4] Suppression of WT1 protein expression by miR-115

After the AZ-521 cells were cultured and trypsinized as described above in Example 3, the cells were adjusted to 1.2 x 10⁵ cells/2 ml and plated in 6-well plates. After 24 hours, the cells were treated with miR-115 or Luciferase AS (conc. 2 µM) in the presence of RNAi Feet (QIAGEN) for 12 hours.

The expression of WT1 protein in the cells was analyzed by Western blotting. The cells were trypsinized and washed twice with PBS. The cells were lysed in Laemmieli's SDS sample buffer at 10⁶ cells/100 µl. The proteins were fractionated by SDS-PAGE, and transferred onto a PVDF membrane. The membrane was treated with 1% gelatin/TBST for blocking. The membrane was allowed to react with the primary antibody anti WT1 C-19 Ab (Santa Cruz Biotechnology, 100:1 1 dilution) or anti-GAPDH Ab (1000:1 1 dilution), and then with each corresponding ALP-labeled secondary antibody. WT1 and GAPDH proteins were detected using the BCIP/NBT kit (Nacalai tesque).

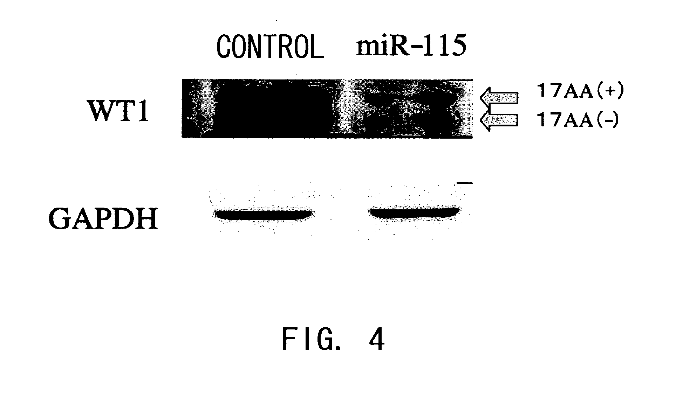

The results are shown in Fig. 4. miR-115 treatment resulted in efficient suppression of the cellular expression of the protein.

### [Example 5] Suppression of the miR-115 effect by forced expression of WT1

The AZ-521 cells in which WT1 was forcedly expressed were treated with miR-115, and the effect of suppressing WT1 expression was examined to confirm whether miR-115 suppresses the growth ofAZ-521 cells in a WT1-specific manner.

The vector pcDNAWT1A(+/+) was prepared by inserting WT1 17AA(+)KTS(+) into the expression vector pcDNA3.1 (Invitrogen) comprising a CMV promoter for forced expression. 2 µg of the vector was introduced into the AZ-521 cells by lipofection using Fugene6 (Roche).

The AZ-521 cells were plated, and after 24 hours, the RNA (final conc. 2 µM) was introduced into the cells using RNAiFect (QIAGEN). After 24 hours, 2 µg of pcDNA3.1/WTA(+/+) or the empty vector was introduced by lipofection. After 72 hours, the cells were harvested and counted. The cell counts were determined using a cytometer after trypsinization.

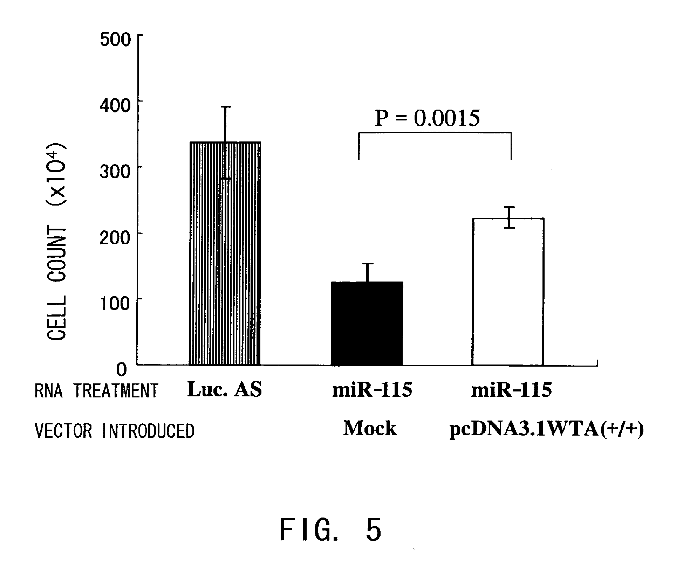

The cell growth-suppressing effect of miR-115 was significantly suppressed in the pcDNA3.1/WTA(+/+)-introduced cells as compared with cells introduced with the empty vector (Fig. 5). These results show that miR-115 suppresses the growth of AZ-521 cells by specifically suppressing the expression of WT1 protein.

### [Example 6] Suppression of the GFP protein by miR115 via a potential miR115target sequence

A vector containing a potential miR115 target sequence inserted on the 3' side of a GFP protein was constructed, and the effect of miR115 on the cellular expression of the GFP protein was analyzed to demonstrate that miR115 suppresses the expression of WT1 protein *via* the potential target sequence of WT1 mRNA.

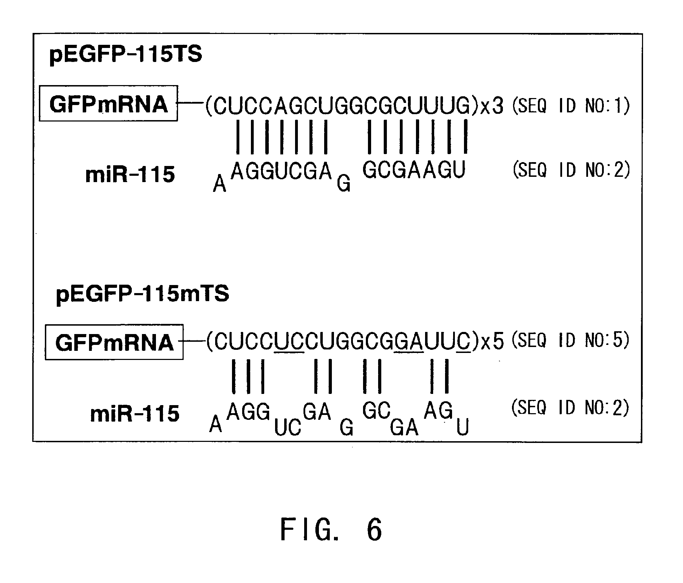

First, an oligo DNA comprising three units of the 17-nucleotide sequence (the potential miR115 target sequence) upstream of the WT1 gene stop codon (2072 nt to 2088 nt; SEQ ID NO: 1), or an oligo DNA comprising five units of a nucleotide sequence (SEQ ID NO: 5) having mutations at five sites of the sequence described above was inserted into a pEGFP-c3 vector (BD) between the *Sca*I and *Eco*RI sites, to construct the pEGFP-115TS vector and pEGFP-115mTS vector, respectively (Fig. 6). Then, the two types of vectors (pEGFP-115TS and pEGFP-115mTS) were linearized by digestion with the restriction enzyme *Alw*44I, and introduced into AZ-521 gastric cancer cells by electroporation. The cells were cultured in a selection medium containing G418 for two weeks or more. Cell lines overexpressing EGFP (AG115TS and AG115mTS, respectively) were established through FACS analysis.

Two types of human miRNA, miR-115: 5'- UGAAGCGGAGCUGGAA- 3' and Let7e: 5'- UGAGGUAGGAGGUUGUAUAGU -3', were synthesized (Japan Bio Service), and the cell lines expressing EGFP were treated with the microRNA. The microRNA treatment was carried out by the following procedure: the cells were plated in 6-well plates the day before, and 2 µM each of miR-115 and Let7e was introduced using the RNAiFect transfection reagent (QIAGEN) the next day. After 24 hours, the cells were harvested and the expression levels of EGFP were analyzed by Western blotting.

Western blotting was performed as follows. The cells treated with miR-115 or Let7e as a control were lysed in SDS sample buffer. The proteins were fractionated by SDS-PAGE, and then transferred onto a PVDF membrane. Anti-GFP monoclonal antibody (Santa Cruz Biotechnology) and anti-GAPDH antibody (Chemicon) were used as primary antibodies, and ALP-conjugated antimouse antibody (Santa Cruz Biotechnology) as a secondary antibody. The color development was achieved using the BCIP-NBT kit.

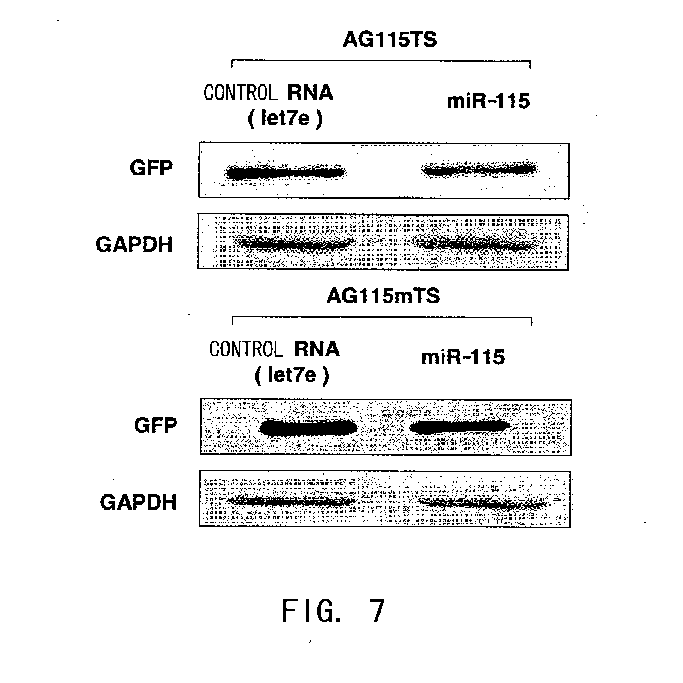

As a result, it was revealed that, when AG115TS cells, which are AZ-521 cells expressing a GFP vector comprising three units of the potential miR115target sequence (17 nucleotides upstream of the WT1 gene stop codon (2072 nt to 2088 nt), were treated with miR115, the expression of the GFP protein decreased; whereas the expression of GFP protein was not altered by the let7e treatment. It was found that the expression of the GFP protein was not reduced by the miR115 treatment in AG115mTS cells, which are AZ-521 cells expressing a GFP vector that comprises five units of the potential miR115 target sequence comprising mutations at five sites (Fig. 7). These results show that miR115 suppresses protein expression *via* the potential target sequence.

### Industrial Applicability

The present invention provides microRNA that can efficiently suppress the expression of WT1 gene, and cell growth-suppressing agents comprising the microRNA as an active ingredient. Since the WT1 gene is known to be expressed at high levels in cancer cells, the cell growth-suppressing agents of the present invention are particularly useful as novel anticancer drugs. It is also known that the overexpression of WT1 suppresses cell differentiation or apoptosis. This suggests that the suppression of WT1 expression by miR115 of the present invention will induce cell differentiation or cell death in cancer cells.

## Claims

1. A cell growth-suppressing agent comprising any one of (a) to (c) as an active ingredient:
(a) a single-stranded RNA complementary to a transcript of WT1 gene;
(b) a DNA encoding the RNA of (a); and
(c) a vector carrying the DNA of (b) as an insert.

2. The cell growth-suppressing agent of claim 1, wherein the single-stranded RNA is complementary to the nucleotide sequence of the WT1 gene transcript shown in SEQ ID NO: 1.

3. The cell growth-suppressing agent of claim 1 or 2, wherein the single-stranded RNA comprises the nucleotide sequence of SEQ ID NO: 2.

4. A cell differentiation-inducing agent or cell death-inducing agent comprising any one of (a) to (c) as an active ingredient:
(a) a double-stranded RNA comprising an RNA complementary to a transcript of WT1 gene and an RNA complementary to the RNA;
(b) a DNA encoding the double-stranded RNA of (a); and
(c) a vector carrying the DNA of (b) as an insert.

5. The agent of claim 4, wherein the single-stranded RNA is complementary to the nucleotide sequence of the WT1 gene transcript shown in SEQ ID NO: 1.

6. The agent of claim 4 or 5, wherein the single-stranded RNA comprises the nucleotide sequence of SEQ ID NO: 2.
